# EUROPÄISCHE PATENTSCHRIFT

(11) **EP 1 958 609 B1**
(45) Veröffentlichungstag und Bekanntmachung des Hinweises auf die Patenterteilung: **09.09.2009**
(21) Anmeldenummer: 07102401.2
(22) Anmeldetag: 14.02.2007
(51) Int. Cl.: A61K 8/06, A61K 8/49, A61K 8/81, A61Q 17/04

(54) **Kosmetische Zubereitung mit wasserlöslichem UV-Lichtschutzfilter und Vinylpyrrolidon/Acrylsäure-Copolymer**
Cosmetic preparation with water-soluble UV filter and vinylpyrrolidone/acrylic acid copolymer
Préparation cosmétique comprenant des filtres UV hydrosolubles et un copolymère vinylpyrrolidone/acide acrylique

(43) Veröffentlichungstag der Anmeldung: 20.08.2008
(73) Patentinhaber: Beiersdorf AG, 20245 Hamburg (DE)
(72) Erfinder: Viala, Sophie, 20257, Hamburg (DE); Clausen, Andreas, 20146, Hamburg (DE); Storbeck, Celina, 25474, Bönniggstedt (DE); Tesch, Mirko, 22303, Hamburg (DE); Skubsch, Kerstin, 25421, Pinneberg (DE)

(56) Entgegenhaltungen:
- EP-A- 1 459 801
- WO-A-20/05046582
- DE-A1- 10 000 209
- DE-A1- 10 108 387
- DE-A1- 10 215 656
- FR-A1- 2 820 030
- US-A- 5 073 614
- US-A1- 2003 147 825

## Beschreibung

Die vorliegende Erfindung betrifft Sonnenschutzmittel mit Vinylpyrrolidon/Acrylsäure-Copolymer.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Zubereitungen, welche die wasserlöslichen UV-Lichtschutzfilter gewählt aus der Gruppe der Verbindungen 2-Phenylbenzimidazol-5-sulfonsäure und ihre Natrium-, Kalium- und Triethanolammoniumsalze bzw. Phenylen-1,4-bis-(2-benzimidazyl)-3,3',5,5'-tetrasulfonsäure und ihre Natrium-, Kalium- und Triethanolammoiumsalze enthalten und dadurch einen hohen Elektrolytgehalt aufweisen, sind nur schwer als Emulsion zu formulieren. Derartige Emulsionen lassen sich nach dem Stand der Technik nur durch die Kombination mehrerer Verdicker oder durch den Einsatz eines für die jeweilige Rezeptur speziell entwickeltes Emulgatorsystems halbwegs stabil herstellen. Die so hergestellten Zubereitungen weisen dann in der Regel unkosmetische Eigenschaften auf. Diese Emulsionen haben dann ein Gries-artiges Aussehen. Bei der Entnahme aus einem Vorratsgefäß bilden sich Fäden und auf der Haut fühlen sie sich klebrig und unangenehm an.

Es war daher die Aufgabe der vorliegenden Erfindung, die Nachteile des Standes der Technik zu beseitigen. Ferner war es die Aufgabe stabile kosmetische Emulsionen (bzw. Dispersionen) mit einem hohen Gehalt an wasserlöslichen UV-Lichtschutzfiltern auf Elektrolytbasis zu entwickeln, welche bei der Anwendung auf der Haut eine ansprechende Sensorik aufweisen.

Überraschend gelöst werden die Aufgaben durch eine kosmetische Zubereitung enthaltend
a) ein oder mehrere wasserlösliche UV-Lichtschutzfilter gewählt aus der Gruppe der Verbindungen 2-Phenylbenzimidazol-5-sulfonsäure und ihre Natrium-, Kalium- und Triethanolammoniumsalze sowie Phenylen-1,4-bis-(2-benzimidazyl)-3,3',5,5'-tetrasulfonsäure und ihre Natrium-, Kalium- und Triethanolammoiumsalze,
b) ein Copolymer aus Vinylpyrrolidon und Acrylsäure.

Die erfindungsgemäßen Zubereitungen lassen sich überraschend mit einer reduzierten Menge an Emulgatoren und/oder Coemulgatoren (z.B. Fettalkohole) stabil formulieren. Teilweise lassen sich Zubereitungen (Emulsionen/Dispersionen) sogar unter Verzicht auf den Einsatz von Coemulgatoren (Fettalkoholen) stabil herstellen.

Es ist erfindungsgemäß vorteilhaft, wenn die erfindungsgemäße Zubereitung die wasserlöslichen UV-Lichtschutzfilter in einer Konzentration von 0,1 bis 20 Gewichts-%, bezogen auf das Gesamtgewicht der Zubereitung, enthält und erfindungsgemäß bevorzugt, wenn die erfindungsgemäße Zubereitung die wasserlöslichen UV-Lichtschutzfilter in einer Konzentration von 0,1 bis 10 Gewichts-%, bezogen auf das Gesamtgewicht der Zubereitung, enthält.

Es ist erfindungsgemäß vorteilhaft, wenn die erfindungsgemäße Zubereitung das Copolymer aus Vinylpyrrolidon und Acrylsäure in einer Konzentration von 0,1 bis 10 Gewichts-% und bevorzugt in einer Konzentration von 0,1 bis 5 Gewichts-%, jeweils bezogen auf das Gesamtgewicht der Zubereitung, enthält.

Erfindungsgemäß vorteilhafte Ausführungsformen der vorliegenden Erfindung sind dadurch gekennzeichnet, dass das Gewichtsverhältnis von wasserlöslichen UV-Lichtschutzfiltern zu Copolymeren von 1 :15 bis 15 :1 beträgt.

Es ist vorteilhaft im Sinne der vorliegenden Erfindung, wenn das erfindungsgemäße Copolymer mit Pentaaerythritoltriallylether quervernetzt ist. In dieser Ausführungsform ist es erfindungsgemäß bevorzugt, wenn das Polymer 25-80 Gew.-% Vinylpyrrolidon, 20-80 Gew.-% Acrylsäure und 0,4-2 Gew.-% Pentaaerythritoltriallylether enthält.

Erfindungsgemäß vorteilhafte Ausführungsformen der vorliegenden Erfindung sind dadurch gekennzeichnet, dass die Zubereitung in Form einer Emulsion, bevorzugt in Form einer O/W-Emulsion vorliegt.

Erfindungsgemäß vorteilhaft beträgt die Konzentration des Emulgatorsystems in der Zubereitung 0,01-6 Gew.-%, bezogen auf das Gesamtgewicht der Zubereiutng.

Erfindungsgemäß vorteilhaft enthält die erfindungsgemäße Zubereitung zusätzlich einen oder mehrere weitere UV-Filter gewählt aus der Gruppe der Verbindungen 1,4-di(2-oxo-10-Sulfo-3-bornylidenmethyl)-Benzol und dessen Salze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenyl-acrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titiandioxid; Zinkoxid in einer Konzentration von 0,01 bis 40 Gewichts-% und bevorzugt in einer Konzentration von 1 bis 30 Gewichts-%, bezogen auf das Gesamtgewicht der Zubereitung.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß vorteilhaft sind die erfindungsgemäßen Zubereitungen frei von p-Methylbenzylidencampher.

Erfindungsgemäß vorteilhafte Ausführungsformen der vorliegenden Erfindung sind dadurch gekennzeichnet, dass die Zubereitung weitere in Wasser lösliche Salze in einer Konzentration von 0,001 bis 10 Gewichts-%, bezogen auf das Gesamtgewicht der Zubereitung, enthält.

Als "in Wasser lösliche Salze" werden erfindungsgemäß Salze verstanden, bei denen sich mindestens 1 g Salz in 100g Wasser bei 20 °C lösen lassen.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Butylenglykol, Caprylylglykol, Hexandiol, Pentylenglykol, Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren.

Erfindungsgemäß vorteilhaft enthält die erfindungsgemäße Zubereitung einen oder mehrere Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen eine Schutzfunktion hat.

Es ist insbesondere von Vorteil, die Filmbildner aus der Gruppe der Polymere auf Basis von Polyvinylpyrrolidon (PVP) 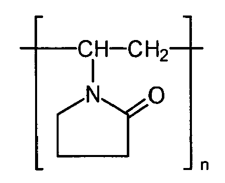 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole. Ebenfall vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann sowie Dermacryl 79 (Acrylates/Octylacrylamide Copolymer) von National Starch.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Myristylmyristat, Isodecylneopentanoat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von Symrise).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin, Cyclometicon, Dimethicon, Phenyltrimethicon und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)

- im Bereich von 1 bis 20 mol-% liegt, wenn das Organopolysiloxan nicht zyklisch ist und
- im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Zum Einen ist es erfindungsgemäß vorteilhaft, wenn die Zubereitung in Form einer O/W-Emulsion vorliegt. In diesem Falle ist es erfindungsgemäß besonders bevorzugt, wenn die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, PEG-100 Stearat, Natriumstearoylglutamat und Natriumcetearylsulfat enthält.

Zum Anderen ist es erfindungsgemäß vorteilhaft, wenn die Zubereitung in Form einer W/O-Emulsion vorliegt. In diesem Falle ist es erfindungsgemäß besonders bevorzugt, wenn die Zubereitung einen oder mehrere W/O-Emulgatoren gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

Die erfindungsgemäßen Zubereitungen können ferner vorteilhaft auch Selbstbräunungssubstanzen enthalten, wie beispielsweise Dihydroxyacteon und/oder Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Hydroxyethylharnstoff, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 30 Gewichts-% und bevorzugt in einer Gesamtkonzentration von 0,5 bis 20 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid) und/oder Licochalcon A.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen) und ermüdete Haut. Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

Zur Anwendung werden die erfindungsgemäßen kosmetischen Zubereitungen in der für Kosmetika üblichen Weise auf die Haut und/oder die Haare in ausreichender Menge aufgebracht.

### Vergleichsversuche

| | | **A** | **B** | **C** | **D** | **E** | **F** |
|---|---|---|---|---|---|---|---|
| **Handelsname** | **Rohstoff** | **m[%]** | **m[%]** | **m[%]** | **m[%]** | **m[%]** | **m[%]** |
| | Polyglyceryl-3 Methylglucose Distearat | 1,5 | 1,5 | 1,5 | 1,5 | 1,5 | 1,5 |
| | Butyl Methoxydibenzoylmethan | 2,0 | 2,0 | 2,0 | 2,0 | 2,0 | 2,0 |
| | Ethylhexyl Salicylate | 5,0 | 5,0 | 5,0 | 5,0 | 5,0 | 5,0 |
| | Phenylbenzimidazolsulfonsäure | | 3,0 | 3,0 | 3,0 | 3,0 | 3,0 |
| | Glycerin | 7,0 | 7,0 | 7,0 | 7,0 | 7,0 | 7,0 |
| | Butylen Glykol Dicaprylat/Dicaprat | 3,0 | 3,0 | 3,0 | 3,0 | 3,0 | 3,0 |
| | Copolymer aus Vinylpyrrolidon und Acrylsäure | 1,6 | 1,6 | | | | |
| Pemulen TR 1 | Acrylates/C10-30 Alkyl Acrylate Crosspolymer | | | | | 1,6 | |
| Aristoflex AVC | Ammonium Acryloyldimethyltaurate/VP Copolymer | | | | 1,6 | | |
| Natrosol Plus 330 CS | Cetyl Hydroxyethylcellulose | | | | | | 1,6 |
| Keltrol F | Xanthan Gum | | | 1,6 | | | |
| | Konservierung | q.s. | q.s. | q.s. | q.s | q.s | q.s. |
| | Neutralisierungsmittel | q.s. | q.s. | q.s. | q.s | q.s | q.s. |
| | Fragrance | q.s. | q.s. | q.s. | q.s | q.s | q.s. |
| | Water | ad 100 | ad 100 | ad 100 | ad 100 | ad 100 | ad 100 |
| | Viskosität in mPa*s bei 25°C (Haake VT02) | > 10000 | 7000 | | 3000 | | |
| | Stabilität | stabil | stabil | in-homogen | stabil | in-homogen | in-homogen |
| | Aussehen + Sensorik | | glatt, homogen, gut verteilbar, angenehmes Hautgefühl | fadenziehend | klebrig | stark griessig | |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### O/W-Emulsionen

| **Emulsion** | **A** | **B** | **C** | **D** | **E** | **F** | **G** | **H** |
|---|---|---|---|---|---|---|---|---|
| Glyceryl Stearat Citrat | 1,5 | 1,5 | 2 | | | | | |
| Sorbitanstearat | | | | | | | 1,0 | |
| Polyglyceryl-3 Methylglycose Distearat | | | | 0,5 | 1,5 | 2,5 | 3,0 | 1,5 |
| Cetearylalkohol | 2 | | | | | | 1,5 | |
| Stearylalkohol | | | 2 | | | 1 | | |
| Cetylalkohol | | | | | | | | 3 |
| Copolymer aus Vinylpyrrolidon und Acrylsäure | 0,5 | 1,5 | 1,0 | 3,0 | 1,6 | 1,5 | 0,8 | 2,0 |
| Acrylates/C10-30 Alkyl Acrylate Crosspolymer | | | 0,3 | | | | | |
| C₁₂₋₁₅ Alkyl Benzoat | | | 5 | | 3 | | | |
| Butylenglycol Dicaprylat/Dicaprat | 10 | | 5 | | 5 | | | |
| Phenethylbenzoat | | | 3 | | | | | |
| Dicaprylyl Ether | | | | | | | | |
| Octyldodekanol | 3 | | | | 3 | | 5 | |
| Dicaprylylcarbonat | | 5 | | | | | | 2 |
| Myristyl Myristat | 1 | | | 2 | | | | |
| Caprylic/Capric Triglycerid | | 2 | | | | | 2 | 3 |
| Isodecyl Neopentanoate | | | | | | | | |
| Isohexadecan | | | | | | 2 | | |
| Cyclomethicon | | | 5 | | | 3 | | 4 |
| Dimethicon | | | | | 2 | | | |
| Butylenglycol | | 2 | | | | | 5 | 8 |
| Glycerin | 2 | 5 | 10 | 5 | 7,5 | 4 | 2 | |
| C18-38 Säuretriglyceride | | | 1 | | | | | |
| Bis-Ethylhexyloxyphenol Methoxyphenyl Triazin | | | 2 | 1 | | | 2 | |
| Butyl Methoxydibenzoylmethan | 2,5 | | 3 | | 2 | | 5 | |
| Diethylamino Hydroxybenzoyl Hexyl Benzoat | | 1 | | 4 | | | | |
| Ethylhexyl Methoxycinnamat | | 5 | | | | | | 7,5 |
| Octylsalicylat | 4 | | | | 5 | | | |
| Benzophenon-3 | | | | | | | | 2 |
| Phenylbenzimidazol Sulfonsäure | 3 | 2 | 1 | 4 | 2 | 1,5 | 4 | 2 |
| Phenylen-1,4-bis-(2-benzimidazyl)-3,3',5,5'-tetrasulfonsäure | | 2 | | | | 2 | | |
| Ethylhexyltriazin | | | 2 | | | 2 | 1 | |
| Diethylhexylbutamidotriazin | | | | | 1 | | | |
| Octodecendicarbonsäure | | | | | | | | 1 |
| Aluminium Stärke Octenylsuccinat | | | | | 1 | | | |
| Natrium Stärke Octenylsuccinat | 3 | | | | | | 1,5 | |
| Tapioka Stärke | | 2 | | | | 1 | 3 | |
| Alkohol | 0,5 | 3 | | | 5 | | | |
| Taurin | 0,1 | 0,2 | | | | 0,5 | 0,2 | |
| Folsäure | 0,01 | | 0,03 | 0,01 | | 0,02 | | 0,03 |
| Vitamin E Acetat | 0,2 | 0,2 | 0,2 | | | 0,3 | 0,1 | 0,5 |
| Na₂H₂EDTA | 0,1 | 0,1 | 0,2 | | | 0,2 | 0,2 | 0,5 |
| Parfüm, Konservierungsmittel | q.s. | q.s. | q.s. | q.s. | q.s. | q.s. | q.s. | q.s. |
| Farbstoffe, usw. | q.s. | q.s. | q.s. | q.s. | q.s. | q.s. | q.s. | q.s. |
| Natriumhydroxid | q.s. | q.s. | q.s. | q.s. | q.s. | q.s. | q.s. | q.s. |
| Wasser | ad 100,0 | ad 100,0 | ad 100,0 | ad 100,0 | ad 100,0 | ad 100,0 | ad 100,0 | ad 100,0 |

### O/W-Emulsionen

| **Emulsion** | **I** | **K** | **L** | **M** | **N** | **O** | **P** | **Q** |
|---|---|---|---|---|---|---|---|---|
| PEG-40 Stearat | 1,5 | 2 | 0,8 | 0,5 | | | | |
| Glyceryl Stearat | | 3,5 | 2,6 | | | | 2 | |
| Natrium Stearoylglutamat | | | | | 0,05 | 0,1 | 0,2 | 0,5 |
| Cetearylalkohol | | | 3 | | | | | |
| Stearylalkohol | | | | | | | 2 | |
| Cetylalkohol | | | | | | | | |
| Copolymer aus Vinylpyrrolidon und Acrylsäure | 1,6 | 0,5 | 1,5 | 3 | 2 | 1,6 | 0,5 | 1 |
| Carbomer | | 0,2 | 0,3 | | | 0,2 | | |
| C₁₂₋₁₅ Alkyl Benzoat | 7 | 3 | 3 | | | 5 | 1 | 10 |
| Butylenglycol Dicaprylat/Dicaprat | 9 | | 3 | | 2 | 8 | | 10 |
| Phenethylbenzoat | 5 | | | | | | 2 | 5 |
| Dicaprylyl Ether | | | | | | | | |
| Octyldodekanol | 3 | | 5 | | | 2 | 4 | |
| Dicaprylylcarbonat | | | | 2 | | | | |
| Myristyl Myristat | | | | | | | | 1 |
| Caprylic/Capric Triglycerid | | | | | | | 2 | |
| Isodecyl Neopentanoate | | | | | 3 | | | |
| Isohexadecan | | 3 | | | | | | |
| Cyclomethicon | | | | 5 | 2 | | | |
| Dimethicon | | 5 | | | 2 | | | |
| Glycerin | 3 | 5 | 5 | 7 | 10 | 2 | 5 | 8 |
| Bis-Ethylhexyloxyphenol Methoxyphenyl Triazin | 3 | | | | | 1 | | 2 |
| Butyl Methoxydibenzoylmethan | 1 | | 2,5 | | | 4 | | 3 |
| Polysilicon-15 | 3 | | | | | | 2 | |
| Ethylhexyl Methoxycinnamat | | 2 | 5 | | | 3 | | |
| Octylsalicylat | | | | 5 | | 3 | 2 | |
| Octocrylen | | | 4 | | | | | |
| Phenylbenzimidazol Sulfonsäure | 4 | 1 | 2 | 3 | 1 | 0,5 | 1 | 2,5 |
| Phenylen-1,4-bis-(2-benzimidazyl)-3,3',5,5'-tetrasulfonsäure | | 2 | | | 1 | | 3 | 0,5 |
| Ethylhexyltriazin | | | | | 2 | | | 3 |
| Titandioxid | | | | 0,5 | 2 | | | |
| Aluminium Stärke Octenylsuccinat | 1 | 5 | | | 2 | | | 2 |
| Natrium Stärke Octenylsuccinat | | | 2 | | | | 3 | |
| Tapioka Stärke | | | | 3 | 2 | | | |
| Alkohol | 2 | | | 5 | | | 4 | |
| Folsäure | 0,03 | 0,01 | 0,03 | | | 0,03 | 0,02 | |
| Q 10 | | | 0,03 | | 0,05 | | | |
| Na₂H₂EDTA | 0,1 | 0,1 | 0,2 | 0,1 | 0,2 | 0,2 | 0,2 | 0,5 |
| Parfüm, | q.s. | q.s. | q.s. | q.s. | q.s. | q.s. | q.s. | q.s. |

| Konservierungsmittel | | | | | | | | |
|---|---|---|---|---|---|---|---|---|
| Farbstoffe, usw. | q.s. | q.s. | q.s. | q.s. | q.s. | q.s. | q.s. | q.s. |
| Natriumhydroxid | q.s. | q.s. | q.s. | q.s. | q.s. | q.s. | q.s. | q.s. |
| Wasser | ad 100,0 | ad 100,0 | ad 100,0 | ad 100,0 | ad 100,0 | ad 100,0 | ad 100,0 | ad 100,0 |

### O/W-Emulsionen

| **Emulsion** | **S** | **T** | **U** | **V** | **W** | **X** | **Y** | **Z** |
|---|---|---|---|---|---|---|---|---|
| Cetearyl Alkohol + PEG-40 Rizinusöl+ Natrium Cetearyl Sulfat | 2,5 | 2,5 | 3 | | | | | |
| Glyceryl Stearat SE | 1 | 1 | 1,5 | | | | | |
| Ceteareth-20 | | | | | 1 | 0,5 | 0,5 | |
| Cetearylalkohol | | | | | | | | |
| Stearylalkohol | | | | | | | | |
| Cetylalkohol | | | | | | | | |
| Acrylates/C10-30 Alkyl Acrylate Crosspolymer | | | 0,3 | | | | 0,5 | 0,3 |
| Copolymer aus Vinylpyrrolidon und Acrylsäure | 1,5 | 1,0 | 2 | 3 | 3 | 4 | 1 | 2,5 |
| C₁₂₋₁₅ Alkyl Benzoat | 10 | 5 | | | 4 | 3 | 2 | 5 |
| Butylenglycol Dicaprylat/Dicaprat | 10 | 5 | 3 | | | | 5 | 8 |
| Phenethylbenzoat | | 5 | | | | | 2 | |
| Dicaprylyl Ether | | | 2 | | | | | |
| Octyldodekanol | 2 | | | 2 | | 1 | | |
| Dicaprylylcarbonat | | 2 | | | 2 | | | |
| Caprylic/Capric Triglycerid | 3 | | | 3 | | | | |
| Isodecyl Neopentanoate | | | 2 | | | | | 3 |
| Cyclomethicon | 4 | | | | 3 | | | |
| Dimethicon | | 3 | | | | | 2 | |
| Glycerin | 5 | 3 | 2 | 5 | 7 | 1 | 8 | 2 |
| VP/Hexadecene Copolymer | 1 | | | | | 1 | | |
| C18-38 Säuretriglyceride | 1 | | | | | 1 | 1 | |
| Bis-Ethylhexyloxyphenol Methoxyphenyl Triazin | 2 | | 1 | | | | | |
| Butyl Methoxydibenzoylmethan | 5 | 1 | | | | 2,5 | 2,5 | 2 |
| Homosalat | | | 5 | | | | 5 | |
| Ethylhexyl Methoxycinnamat | | 5 | | 8 | 5 | | 5 | |
| Diethylamino Hydroxybenzoyl Hexyl Benzoat | | 3 | | | 2 | | | |
| Octocrylen | 8 | | 5 | 5 | | | | |
| Octylsalicylat | | | | | | 5 | 3 | |
| Phenylbenzimidazol Sulfonsäure | 3 | 2 | 2 | 2 | 1 | 3 | 2 | 2 |
| Phenylen-1,4-bis-(2-benzimidazyl)-3,3',5,5'-tetrasulfonsäure | | 1 | 2 | 2 | | 3 | 2,5 | |
| Ethylhexyltriazin | 3 | 2 | | | | | | 2 |
| Diethylhexylbutamidotriazin | | 2 | | | | | | |
| Benzophenon-3 | | | | | | | 2 | |
| 2,4-bis-[5-1 (dimethyl-propyl)benzoxazol-2-yl-(4-phenyl)-imino]-6-(2-ethylhexyl)-imino-1,3,5-triazin | | | | 3 | 1 | | | |
| Titandioxid | 5 | | 3 | | | | | |
| Aluminium Stärke Octenylsuccinat | | 2 | | | 0,5 | | | |
| Natrium Stärke Octenylsuccinat | 1,5 | | 1 | | | 0,5 | | |
| Tapioka Stärke | 3 | | | | | | | 1 |
| Alkohol | 3 | 5 | 4 | | | 4 | | 2 |
| Taurin | 0,5 | | 0,2 | | 0,6 | | 1 | 0,1 |
| Vitamin E | 1 | 0,2 | | 0,5 | | 0,3 | | 0,5 |
| Na₂H₂EDTA | 0,1 | 0,1 | 0,2 | 0,1 | 0,1 | 0,2 | 0,2 | 0,5 |
| Parfüm, Konservierungsmittel | q.s. | q.s. | q.s. | q.s. | q.s. | q.s. | q.s. | q.s. |
| Farbstoffe, usw. | q.s. | q.s. | q.s. | q.s. | q.s. | q.s. | q.s. | q.s. |
| Natriumhydroxid | q.s. | q.s. | q.s. | q.s. | q.s. | q.s. | q.s. | q.s. |
| Wasser | ad 100,0 | ad 100,0 | ad 100,0 | ad 100,0 | ad 100,0 | ad 100,0 | ad 100,0 | ad 100,0 |

## Patentansprüche

1. Kosmetische Zubereitung enthaltend
a) ein oder mehrere wasserlösliche UV-Lichtschutzfilter gewählt aus der Gruppe der Verbindungen 2-Phenylbenzimidazol-5-sulfonsäure und ihre Natrium-, Kalium- und Triethanolammoniumsalze sowie Phenylen-1,4-bis-(2-benzimidazyl)-3,3',5,5'-tetrasulfonsäure und ihre Natrium-, Kalium- und Triethanolammoiumsalze,
b) ein Copolymer aus Vinylpyrrolidon und Acrylsäure.

2. Kosmetische Zubereitung nach Anspruch 1, **dadurch gekennzeichnet, dass** die Zubereitung die wasserlöslichen UV-Lichtschutzfilter in einer Konzentration von 0,1 bis 20 Gewichts-%, bezogen auf das Gesamtgewicht der Zubereitung, enthält.

3. Kosmetische Zubereitung nach einem der vorhergehenden Ansprüche, **dadurch gekennzeichnet, dass** die Zubereitung das Copolymer aus Vinylpyrrolidon und Acrylsäure in einer Konzentration 0,1 bis 10 Gewichts-%, bezogen auf das Gesamtgewicht der Zubereitung, enthält.

4. Kosmetische Zubereitung nach einem der vorhergehenden Ansprüche, **dadurch gekennzeichnet, dass** das Gewichtsverhältnis von wasserlöslichen UV-Lichtschutzfiltern zu Copolymeren von 1:15 bis 15:1 beträgt.

5. Kosmetische Zubereitung nach einem der vorhergehenden Ansprüche, **dadurch gekennzeichnet, dass** das Copolymer mit Pentaaerythritoltriallylether quervernetzt ist.

6. Kosmetische Zubereitung nach einem der vorhergehenden Ansprüche, **dadurch gekennzeichnet, dass** das Polymer 25-80 Gew.-% Vinylpyrrolidon, 20-80 Gew.-% Acrylsäure und 0,4-2 Gew.-% Pentaaerythritoltriallylether enthält.

7. Kosmetische Zubereitung nach einem der vorhergehenden Ansprüche, **dadurch gekennzeichnet, dass** die Zubereitung in Form einer Emulsion vorliegt.

8. Kosmetische Zubereitung nach einem der vorhergehenden Ansprüche, **dadurch gekennzeichnet, dass** die Zubereitung zusätzlich einen oder mehrere weitere UV-Filter gewählt aus der Gruppe der Verbindungen 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester 4-(tert.-Butyl-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan-Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titiandioxid; Zinkoxid in einer Konzentration von 0,01 bis 40 Gewichts-%, bezogen auf das Gesamtgewicht der Zubereitung, enthält.

9. Zubereitung nach einem der vorhergehenden Ansprüche, **dadurch gekennzeichnet, dass** die Zubereitung weitere in Wasser lösliche Salze in einer Konzentration von 0,001 bis 10 Gewichts-%, bezogen auf das Gesamtgewicht der Zubereitung, enthält.

10. Zubereitung nach einem der vorhergehenden Ansprüche, **dadurch gekennzeichnet, dass** die Zubereitung frei ist von Fettalkoholen.

## Claims

1. Cosmetic preparation comprising
a) one or more water-soluble UV photoprotective filters selected from the group of the compounds 2-phenylbenzimidazole-5-sulphonic acid and its sodium, potassium and triethanolammonium salts and also phenylene-1,4-bis(2-benzimidazyl)-3,3',5,5'-tetrasulphonic acid and its sodium, potassium and triethanolammonium salts,
b) a copolymer of vinylpyrrolidone and acrylic acid.

2. Cosmetic preparation according to Claim 1, **characterized in that** the preparation comprises the water-soluble UV photoprotective filters in a concentration of from 0.1 to 20% by weight, based on the total weight of the preparation.

3. Cosmetic preparation according to one of the preceding claims, **characterized in that** the preparation comprises the copolymer of vinylpyrrolidone and acrylic acid in a concentration of from 0.1 to 10% by weight, based on the total weight of the preparation.

4. Cosmetic preparation according to one of the preceding claims, **characterized in that** the weight ratio of water-soluble UV photoprotective filters to copolymers is from 1:15 to 15:1.

5. Cosmetic preparation according to one of the preceding claims, **characterized in that** the copolymer is crosslinked with pentaerythritol triallyl ether.

6. Cosmetic preparation according to one of the preceding claims, **characterized in that** the polymer comprises 25-80% by weight of vinylpyrrolidone, 20-80% by weight of acrylic acid and 0.4-2% by weight of pentaerythritol triallyl ether.

7. Cosmetic preparation according to one of the preceding claims, **characterized in that** the preparation is in the form of an emulsion.

8. Cosmetic preparation according to one of the preceding claims, **characterized in that** the preparation additionally comprises one or more further UV filters selected from the group of the compounds 1,4-di(2-oxo-10-sulpho-3-bomylidenemethyl)benzene and salts thereof; 4-(2-oxo-3-bomylidenemethyl)benzenesulphonic acid salts; 2-methyl-5-(2-oxo-3-bomylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxo]-disiloxanyl]propyl]phenol; 3-(4-methylbenzylidenexamphor; 3-benzylidenecamphor; ethylhexyl salicylate; terephthalidenedicamphorsulphonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate, 4-(tert-butyl)-4'-methoxydibenzoylmethane; homomenthyl salicylate; 2-ethylhexyl 2-hydroxybenzoate; 2-ethylhexyl 2-cyano-3,3-diphenylacrylate; dimethicodiethylbenzalmalonate; 3-(4-(2,2-bisethoxycarbonylvinyl)-phenoxy)propenyl)methoxysiloxane/dimethylsiloxane copolymer; 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); dioctylbutylamidotriazone (INCI: Diethylhexyl-Butamidotriazone); 2,4-bis[5-1-(dimethylpropyl)benzoxazol-2-yl-(4-phenyl)imino]-6-(2-ethylhexyl)imino-1,3,5-triazine with the CAS No. 288254-16-0; tris(2-ethylhexyl) 4,4',4"-(1,3,5-triazine-2,4,6-triyltriimino)tris benzoate (also: 2,4,6-tris[anilino(p-carbo-2'-ethyl-1'-hexyloxy)]-1,3,5-triazine (INCI: Ethylhexyl Triazone); 2,4,6-tribiphenyl-4-yl-1,3,5-triazine; merocyanines; titanium dioxide; zinc oxide in a concentration of from 0.01 to 40% by weight, based on the total weight of the preparation.

9. Preparation according to one of the preceding claims, **characterized in that** the preparation comprises further water-soluble salts in a concentration of from 0.001 to 10% by weight, based on the total weight of the preparation.

10. Preparation according to one of the preceding claims, **characterized in that** the preparation is free from fatty alcohols.

## Revendications

1. Préparation cosmétique contenant
a) un ou plusieurs filtres de protection contre la lumière UV solubles dans l'eau, choisis dans le groupe formé par les composés acide 2-phénylbenzimidazole-5-sulfonique et leurs sels de sodium, de potassium et de triéthanolammonium ainsi que l'acide phénylène-1,4-bis-(2-benzimidazyl)-3,3',5,5'-tétrasulfonique et leurs sels de sodium, de potassium et de triéthanolammonium,
b) un copolymère de vinylpyrrolidone et d'acide acrylique.

2. Préparation cosmétique selon la revendication 1, **caractérisée en ce que** la préparation contient les filtres de protection contre la lumière UV solubles dans l'eau en une concentration de 0,1 à 20% en poids, par rapport au poids total de la préparation.

3. Préparation cosmétique selon l'une quelconque des revendications précédentes, **caractérisée en ce que** la préparation contient le copolymère de vinylpyrrolidone et d'acide acrylique en une concentration de 0,1 à 10% en poids, par rapport au poids total de la préparation.

4. Préparation cosmétique selon l'une quelconque des revendications précédentes, **caractérisée en ce que** le rapport pondéral des filtres de protection contre la lumière UV solubles dans l'eau aux copolymères est de 1:15 à 15:1.

5. Préparation cosmétique selon l'une quelconque des revendications précédentes, **caractérisée en ce que** le copolymère est réticulé transversalement avec du pentaérythritoltriallyléther.

6. Préparation cosmétique selon l'une quelconque des revendications précédentes, **caractérisée en ce que** le polymère contient 25-80% en poids de vinylpyrrolidone, 20-80% en poids d'acide acrylique et 0,4-2% en poids de pentaérythritoltriallyléther.

7. Préparation cosmétique selon l'une quelconque des revendications précédentes, **caractérisée en ce que** la préparation se trouve sous forme d'une émulsion.

8. Préparation cosmétique selon l'une quelconque des revendications précédentes, **caractérisée en ce que** la préparation contient en outre un ou plusieurs autres filtres des UV choisis dans le groupe formé par les composés 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylènebis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque ; 4-(tert-butyl)-4'-méthoxydibenzoylméthane ; salicylate d'homomenthyle ; 2-hydroxybenzoate de 2-éthylhexyle ; acrylate de 2-éthylhexyl-2-cyano-3,3-diphényle ; benzalmalonate de diméthicodiéthyle ; copolymère de 3-(4-(2,2-bis-(éthoxycarbonylvinyl)-phénoxy)propényl)-méthoxysiloxane/diméthylsiloxane ; 2,4-bis-{[4-(2-éthylhexyloxy)-2-hydroxy]-phényl}-6-(4-méthoxyphényl)-1,3,5-triazine (INCI : bis-Ethylhexyloxyphenol Methoxyphenyl Triazine) ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (CAS Nr. 288254-16-0) ; ester tris(éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine) (INCI : Ethylhexyl Triazone) ; 2,4,6-tribiphényl-4-yl-1,3,5-triazine ; Merocyanine ; dioxyde de titane ; oxyde de zinc en une concentration de 0,01 à 40% en poids, par rapport au poids total de la préparation.

9. Préparation selon l'une quelconque des revendications précédentes, **caractérisée en ce que** la préparation contient d'autres sels solubles dans l'eau en une concentration de 0,001 à 10% en poids, par rapport au poids total de la préparation.

10. Préparation selon l'une quelconque des revendications précédentes, **caractérisée en ce que** la préparation est exempte d'alcools gras.
